(19) Europäisches Patentamt / European Patent Office / Office européen des brevets

(11) **EP 3 342 417 B1**

(12) **EUROPEAN PATENT SPECIFICATION**

(45) Date of publication and mention of the grant of the patent:
**18.08.2021 Bulletin 2021/33**

(21) Application number: **16838244.8**

(22) Date of filing: **16.03.2016**

(51) Int Cl.:
*A61K 36/752* *(2006.01)*    *A61K 36/62* *(2006.01)*
*A61K 8/97* *(2017.01)*      *A61P 17/04* *(2006.01)*
*A61P 17/00* *(2006.01)*     *A61P 3/04* *(2006.01)*
*A61Q 19/00* *(2006.01)*

(86) International application number:
**PCT/CN2016/076485**

(87) International publication number:
**WO 2017/031981 (02.03.2017 Gazette 2017/09)**

(54) **COMPOSITION FOR BODY SLIMMING AND SKIN CARE AND PREPARATION METHOD THEREOF**

ZUSAMMENSETZUNG FÜR KÖRPERSTRAFFUNG UND HAUTPFLEGE SOWIE HERSTELLUNGSVERFAHREN DAFÜR

COMPOSITION POUR L'AMINCISSEMENT DU CORPS ET LE SOIN DE LA PEAU ET PROCÉDÉ DE PRÉPARATION ASSOCIÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.08.2015 CN 201510527307**

(43) Date of publication of application:
**04.07.2018 Bulletin 2018/27**

(73) Proprietor: **Infinitus (China) Company Ltd.**
**Jiang Men, Guangdong 529100 (CN)**

(72) Inventor: **LIU, Xiaoying**
**Jiangmen**
**Guangdong 529100 (CN)**

(74) Representative: **Kolster Oy Ab**
**(Salmisaarenaukio 1)**
**P.O. Box 204**
**00181 Helsinki (FI)**

(56) References cited:
**CN-A- 104 224 603    US-B1- 6 464 991**

• **DATABASE WPI Week 201540 Thomson Scientific, London, GB; AN 2015-320027 XP002787895, & CN 104 473 045 A (MINGGUANG BAIYUN FLOUR MILLS CO LTD) 1 April 2015 (2015-04-01)**
• **DATABASE WPI Week 201520 Thomson Scientific, London, GB; AN 2015-18368R XP002787896, & CN 104 286 658 A (LIU S) 21 January 2015 (2015-01-21)**
• **DATABASE WPI Week 201437 Thomson Scientific, London, GB; AN 2014-H44052 XP002787897, & CN 103 621 993 A (ZHEJIANG JINSHANMEI BIOLOGICAL TECHNOLOG) 12 March 2014 (2014-03-12)**
• **DATABASE WPI Week 201522 Thomson Scientific, London, GB; AN 2015-18368T XP002787898, & CN 104 286 657 A (ZHAO J) 21 January 2015 (2015-01-21)**
• **LIU, LI: "Cream of Traditional Chinese Herbal Cuisine: a Complete Collection of Herbal Cuisine for Cosmetology", CHINA MEDICAL SCIENCE PRESS, 30 April 2012 (2012-04-30), page 62, XP009510249,**
• **WANG, JIANXIN: "Cosmetic Plant Raw Materials Manual", CHEMICAL INDUSTRY PRESS, 30 June 2009 (2009-06-30), pages 323-324, XP009509166,**
• **ZHANG, LIPING;: "New Research Progress On Tangerine Peel", GUANGMING JOURNAL OF CHINESE MEDICINE, vol. 20, no. 1, 28 February 2005 (2005-02-28), page 41, XP009509161,**

## Description

### Field

The present invention relates to the field of biotechnology, specifically to a composition, preparation method and use thereof, and a skin care product containing the composition and preparation method thereof.

### Background

With the increase of people's living standard, obesity has become one of the factors that affect the quality of people's life, especially for females. Stress from work and life, irregular diet, pregnancy and parturition, lack of exercise and so on become the main causes of obesity. Obesity not only affects the appearance, but also affects the body health.

At present, there are many products for eliminating obesity, including orally administered drugs and external preparations. There are two categories of commercially available weight-loss medicines: appetite suppressant that acts on the central nervous system and pancreatic lipase inhibitor. Appetite suppressant is limited in use due to its possibility of causing adverse reactions to the nervous system. Pancreatic lipase inhibitor Orlistat can help the weight loss through suppressing the decomposition and absorption of fat from foods by inhibiting the activity of pancreatic lipase. But it may cause steatorrhea and the deficiency of fat-soluble vitamin. Recently, it is also reported that Orlistat may cause damage to liver function. Although appetite suppressants lorcaserin and Qsymia have become the first weight-loss medicines authorized by FDA in 13 years, there are still some safety uncertainties about these medicines to the brain, cardiovascular system and so on. Thus, there is almost no weight-loss medicine with good efficacy and no adverse reactions on the market.

External weight-loss preparations, such as slimming cream and body shaping cream are in hot discussion. However, the efficacy of external preparations such as slimming creams on the market is not optimistic. Some products mainly rely on capsaicin to bring "hot" feeling to the skin, thereby causing consumers to mistakenly believe that the fat is burning. Other products achieve the effect of slimming through promoting the perspiration of skin and relieving edema. Due to the feature "cure the symptoms, not the disease" of these products, the weight-loss effect of these products only lasts for a short time and is easily to rebound. What is more, it may cause injury to the skin, leading to side-effect like flaccid skin, aging and so on.

Thereby, it is important to develop an external skin care preparation with effective and lasting slimming effect.

### Summary

In view of above, the present disclosure provides a composition, preparation method and use thereof, and a skin care product containing the composition and preparation method thereof. The composition or skin care product is safe and non-irritating for the skin. The skin care composition and preparation obtained not only increase the secretion level of leptin and accelerate the metabolism of body fat, but also increase the content of collagen in the body, improve skin flaccid and edema caused by lipid-lowering, therefore achieving the synergetic effect of health and slimming. In addition, experiments show that the present disclosure has effect of skin repair, blood circulation improvement and anti-fatigue.

In order to achieve above objectives, the present disclosure provides technical solutions as follows:

The present disclosure provides a composition, comprising the following raw materials in parts by mass:

| Oats | 5-10 parts |
| Bitter orange flowers | 1-3 parts |
| Lotus leaves | 1-3 parts |
| Dried orange peels | 3-5 parts |

The present disclosure also provides a method for preparing the composition, comprising: pulverizing the desired amount of raw materials according to the formula, extracting with water; the temperature for water extraction is from 61°C to 100°C, the duration of water extraction is from 1h to 4h, the mass ratio of the raw materials to water is from 1:21 to 1:40.

Preferably, the temperature for water extraction in the preparation method is from 80°C to 100°C and the duration of water extraction is from 1h to 4h.

Preferably, the mass ratio of the raw materials to water in the preparation method is from 1:30 to 1:40.

Preferably, the preparation method also comprises steps of cooling, filtration and filtrate collection after water extraction.

Preferably, the filtration of the preparation method comprises common filtration and vacuum filtration.

More preferably, the common filtration is to pass 100-mesh to 200-mesh sieve.

Preferably, vacuum filtration is performed in a Buchner funnel having filter papers with 0.3cm to 0.6cm of diatomite in between.

Preferably, vacuum filtration is performed in a Buchner funnel having two layers of filter paper with 0.3cm to 0.6cm of diatomite in between.

Preferably, the filtration process is: cooling extract solution and passing it through 100-mesh sieve; performing vacuum filtration after the residue is separated out.

Preferably, the cooling in the preparation method is cooling a solution to 20°C~30°C.

The present disclosure also provides a composition prepared by the preparation method.

According to the "holistic, dialectical, comprehensive" theory in tradition Chinese medicine, natural botanical functional raw materials are used to increase the collagen content in the body, improve skin flaccid and edema caused by lipid-lowering, therefore achieving the aim of assisting body slimming. In addition, through ELISA method, study on the effect of the composition on leptin secreted by adipocytes shows that secretion of leptin is increased under the synergistic effect of the components. Furthermore, the components of the raw materials have effect on meridian dredging, qi-blood activating and providing qi-blood nutrition to fascia and muscle. Therefore, a slimming composition that has effect on lipometabolism regulation and anti-fatigue is obtained in view of the above.

The present disclosure also provides use of the composition and/or the composition prepared by the preparation method in the manufacture of a medicine, food, health care product and/or skin care product for slimming and skin care.

The present disclosure also provides use of the composition and/or the composition prepared by the preparation method in the manufacture of a medicine, food, health care product and/or skin care product for improving secretion of leptin by adipocyte, treating pruritus, repairing skin, removing redness and swelling, anti-fatigue, promoting blood circulation and promoting collagen synthesis.

The present disclosure also provides a skin care product, comprising the composition and/or the composition prepared by the preparation method and adjuvants acceptable in skin care products.

In some embodiments of the present disclosure, the dosage form of the skin care product can be paste, cream, essence, toner, emulsion or spray.

The present disclosure also provides a method for preparing the skin care product, which is made from the raw materials as follows:

| Phases | INCI Name | Percentage (wt%) |
|---|---|---|
| Phase A | Cyclotetrasiloxane | 0.5~3 wt% |
| | Cyclopentasiloxane | 0.5~3 wt% |
| | Hydrogenated Polyisobutene | 1~5wt% |
| | Arachidyl Alcohol | 0.5~3wt% |
| | Behenyl Alcohol | |
| | Arachidyl Glucoside | |
| | Cetearyl Alcohol | 0.01~1wt% |
| Phase B | Water | As balance |
| | Glycerin | 1~5 wt% |
| | PEG-8 | 1~5wt% |
| | Butanediol | 1~5 wt% |
| | Dimethicone | 0.5~3wt% |
| | Caprylyl Methicone | 0.5~3wt% |
| | Acrylates/C10-30 alkyl acrylate crosspolymer | 0.2~2wt% |
| | Niacinamide | 0.1~2wt% |
| | Allantoin | 0.05~1 wt% |
| Phase C | Hydroxyethyl Acrylate/Sodium Acryloyldimethyl Taurate Copolymer/ Squalane/Water/Polysorbate 60/Sorbitan Isostearate | 0.1~3wt% |

(continued)

| Phases | INCI Name | Percentage (wt%) |
|---|---|---|
| Phase D | Aminomethyl Propanol | Adjust the pH to 5.0~6.5 |
| Phase E | CafeisilaneC | 1~6wt% |
| | Composition of claims 1 or 2 or 8 | 1~10wt% |
| | Chondrus Crispus | 0.5~10wt% |
| Phase F | Phenoxyethanol | 0.2~1.1wt% |
| | Lavender Oil | 0.005~2 wt% |

The preparation method comprises steps as follows:

Step 1: heating and stirring Phase A to obtain a first product;
Step 2: heating and stirring Phase B to obtain a second product;
Step 3: mixing the first product and the second product together and emulsifying the mixture thereof to obtain a third product;
Step 4: mixing Phase C with the third product to obtain a fourth product;
Step 5: stirring and cooling the fourth product; adding Phase D, Phase E and Phase F; mixing and cooling to obtain a product.

Preferably, in the preparation method, the heating temperature in Step 1 is from 80°C to 85°C; the heating rate is from 1°C/min to 2°C/min; the heating duration is from 30min to 35min; the stirring rate in Step 1 is from 20r/min to 60r/min.
Preferably, in the preparation method, the heating temperature in Step 2 is from 80°C to 85°C; the heating rate is from 1°C/min to 2°C/min; the heating duration is from 30min to 35min; the stirring rate in Step 1 is from 20r/min to 60r/min.
Preferably, in the preparation method, the stirring rate of the mixing in Step 3 is from 30r/min to 50r/min; the emulsification is a homogenous emulsification processed under a condition of 2500r/min to 3500r/min for 3min to 5min.
Preferably, in the preparation method, the stirring rate of the mixing in Step 4 is from 30r/min to 50r/min.
Preferably, in the preparation method, the stirring rate in Step 5 is from 30r/min to 50r/min; the cooling temperature is from 40°C to 50°C; the cooling rate is from 1°C/min to 2°C/min.
Preferably, in the preparation method, the cooling temperature in Step 5 is from 30°C to 40°C.
Preferably, the skin care product provided by the present disclosure is prepared by the method as follows:

| Phases | INCI Name | Percentage (wt%) |
|---|---|---|
| Phase A | Cyclotetrasiloxane | 0.5~3 wt% |
| | Cyclopentasiloxane | 0.5~3 wt% |
| | Hydrogenated Polyisobutene | 1~5wt% |
| | Arachidyl Alcohol | 0.5~3wt% |
| | Behenyl Alcohol | |
| | ArachidylGlucoside | |
| | Cetearyl Alcohol | 0.01~1wt% |

(continued)

| Phases | INCI Name | Percentage (wt%) |
|---|---|---|
| Phase B | Water | As balance |
| | Glycerin | 1~5 wt% |
| | PEG-8 | 1~5wt% |
| | Butanediol | 1~5 wt% |
| | Dimethicone | 0.5~3wt% |
| | Caprylyl Methicone | 0.5~3wt% |
| | Acrylates/C10-30 alkyl acrylate crosspolymer | 0.2~2wt% |
| | Niacinamide | 0.1~2wt% |
| | Allantoin | 0.05~1 wt% |
| Phase C | Hydroxyethyl Acrylate/Sodium AcryloyldimethylTaurate Copolymer/ Squalane/ Water/Polysorbate 60/Sorbitan Isostearate | 0.1~3wt% |
| Phase D | Aminomethyl Propanol | Adjust the pH to 5.0~6.5 |
| Phase E | Cafeisilane C | 1~6wt% |
| | Composition of claims 1 or 2 or 8 | 1~10wt% |
| | Chondrus Crispus | 0.5~10wt% |
| Phase F | Phenoxyethanol | 0.2~1.1wt% |
| | Lavender Oil | 0.005~2 wt% |

(1) Phase A is added into an oil phase pot and heated to 80°C~85°C with stirring. The stirring rate is from 20r/min to 60r/min and the heating rate is from 1°C/min to 2°C/min. The temperature is maintained constantly for 30min to 35min until Phase A is dissolved absolutely.

(2) Phase B is added into an aqueous phase pot and heated to 80°C~85°C with stirring (the stirring rate is from 20r/min to 60r/min; the heating rate is from 1°C/min to 2°C/min). The temperature is maintained constantly for 30min to 35min.

(3) Emulsification: under condition of stirring (the stirring rate is from 30r/min to 50r/min), the Phase A in the oil phase pot is added into an emulsifying pot first, and then the Phase B is added into the emulsifying pot. Homogenous emulsification is performed under a condition of 2500r/min to 3500r/min for 3min to 5min.

(4) Phase C is added into the emulsifying pot under stirring, and the stirring rate is from 30r/min to 50r/min.

(5) Under stirring with a stirring rate of 30r/min to 50r/min, the temperature is cooled to 40°C~50°C with a cooling rate from 1°C/min to 2°C/min; Phase D, E and F are added in turns and stirred until evenly.

(6) Mixture is stirred and cooled to below 40°C; a product is obtained through discharge.

The present disclosure also provides a skin care product prepared by the preparation method.

In some embodiments of the present disclosure, a slimming cream is prepared by the preparation method. Using the composition in the present disclosure and the ordinary techniques and adjuvants in skin care product field, other dosage form such as essence, toner, emulsion, spray and so on can also be prepared.

Experiments show that, the composition and/or the composition prepared by the method provided by the present disclosure has effects of improving secretion of leptin by adipocyte, treating pruritus, repairing skin, removing redness and swelling, anti-fatigue, promoting blood circulation and promoting collagen synthesis.

In conclusion, the composition and/or the composition prepared by the method provided by the present disclosure have functions of slimming and skin care.

The recommended method for using the skin care product provided by the present invention is: both the composition and the cream of the present invention can be applied on the skin surface of human body, such as shank, forearm and other part in need for slimming; gently massage until it is absorbed.

Experiments demonstrate that the composition is safe and causes no irritant to skin. The skin care composition and preparation not only increase the secretion level of leptin, accelerate the metabolism of body fat, but also improve the content of collagen in the body, improve skin flaccid and edema caused by lipid-lowering, therefore achieving the aim of healthy slimming and synergistic effect. In addition, experiments show that the present disclosure also has effect on repairing skin and promoting blood circulation, so it can achieve the effects of body slimming as well as skin improvement.

**Brief Description of Drawings**

In order to describe the technical solutions in the examples of the present disclosure or in the prior art more clearly, the accompanying drawings used in description of the embodiments or the prior art will be illustrated briefly.

Figure 1 shows the infrared thermography of shank administrated with the composition of Example 1; 1(A) shows the image before use and 1(B) shows the image after use.
Figure 2 shows the infrared thermography of forearm administrated with the composition of Example 1; 2(A) shows the image before use and 2(B) shows the image after use.
Figure 3 shows the infrared thermography of shank administrated with the composition of Example 4; 3(A) shows the image before use and 3(B) shows the image after use.
Figure 4 shows the infrared thermography of shank administrated with the composition of Example 4; 4(A) shows the image before use and 4(B) shows the image after use.

**Detailed Description**

The present disclosure discloses a composition, preparation method and use thereof, a skin care product containing the composition and the preparation method thereof.

All raw materials in the examples provided by the present disclosure are commercially available. The sources of the raw materials used in the present disclosure are shown in Table 1; the manufacturers and the brands of the equipment used in the present disclosure are shown in Table 2.

**Table 1 Raw materials and sources**

| Phases | INCI Name | Percentage (%) | Manufacturer |
|---|---|---|---|
| Phase A | Cyclotetrasiloxane | 0.5~3% | Momentive Performance Materials, America |
| | Cyclopentasiloxane | 0.5~3% | |
| | Hydrogenated Polyisobutene | 1~5% | Nippon Oil Corporation, Japan |
| | ArachidylAlcohol/Behenyl Alcohol/ArachidylGlucoside MONTANOV 202 (the trade name of ArachidylAlcohol, Behenyl Alcohol, ArachidylGlucoside) | 0.5~3% | SEPPIC, France |
| | Cetearyl Alcohol | 0.01~1% | Corning |
| Phase B | Water | To 100 | Deionized water, laboratory prepared |
| | Glycerin | 1~5% | SumiAsih, Malaysia |
| | PEG-8 | 1~5% | DOW CHEMICAL |
| | Butanediol | 1~5% | CELANESS, America |
| | Dimethicone | 0.5~3% | Dow Corning |
| | Caprylyl Methicone | 0.5~3% | Momentive Performance Materials, America |
| | Acrylates/C10-30 alkyl acrylate crosspolymer | 0.2~2% | Lubrizol Corporation |
| | Niacinamide | 0.1~2% | DSM or Lonza |
| | Allantoin | 0.05~1% | Kunshan Shuangyou Daily Chemical Co., Ltd. |

(continued)

| Phases | INCI Name | Percentage (%) | Manufacturer |
|---|---|---|---|
| Phase C | Hydroxyethyl Acrylate/Sodium Acryloyldimethyl Taurate Copolymer/ Squalane/Water/Polysorbate 60/Sorbitan Isostearate | 0.1~3% | SEPPIC, France |
| Phase D | Aminomethyl Propanol | Adjust the pH to 5.0~6.5 | DOW CHEMICAL |
| Phase E | Cafeisilane C | 1~6% | EXSYMOL, France |
| | Botany Extract | 1~10% | Self-made |
| | Chondrus Crispus | 0.5~10% | BASF Co., Ltd |
| Phase F | Phenoxyethanol | 0.2~1.1% | Schülke International |
| | Lavender Oil | 0.005~2% | Lebermuth Company, America |

**Table 2 Equipment and manufacturer**

| Name | Type | Manufacturer |
|---|---|---|
| Homoeothermic water bath | HH·S1-M | Beijing Changan Scientific Instrument Factory |
| Intelligent Temperature Controlling Heating Stirrer | SZCL | Gongyi City, Yuhua Instrument Co., Ltd. |
| Water Circulation Type Vacuum Pump | SHB-III | Zhengzhou Great Wall Scientific Industrial and Trade Co., Ltd. |

**Examples**

The present disclosure is further illustrated in combination with the examples below.

**Example 1 Preparation of the botanical composition**

(1) The following raw materials were pulverized and then weighed according to the proportions, followed by mixing evenly.
50g of oats, 20g of bitter orange flowers, 20g of lotus leaves, 50g of dried orange peels

(2) Water was added and extraction was carried out. The mass ratio of the raw material to water is 1:21. Extraction was carried out at 61°C for 3h.

(3) The extract solution obtained in Step (2) was cooled to 20°C, filtered through 100-mesh sieve, and subjected to vacuum filtration after the residue was separated out. The extract solution was collected to obtain the composition.

In the vacuum filtration of Step (3), two layers of filter papers were paved in the Buchner funnel with 0.3cm to 0.6cm of diatomite in between.

**Example 2 Preparation of the botanical composition**

(1) The following raw materials were pulverized and then weighed according to the proportions, followed by mixing evenly.

65g of oats, 30g of bitter orange flowers, 10g of lotus leaves, 30g of dried orange peels

(2) Water was added and extraction was carried out. The mass ratio of the raw material to water is 1:30. Extraction was carried out at 100°C for 1h.

(3) The extract solution obtained in Step (2) was cooled to 25°C, filtered through 200-mesh sieve, and subjected to vacuum filtration after the residue was separated out. The extract solution was collected to obtain the composition.

In the vacuum filtration of Step (3), two layers of filter papers were paved in the Buchner funnel with 0.3cm to 0.6cm of diatomite in between.

## Example 3 Preparation of the botanical composition

(1) The following raw materials were pulverized and then weighed according to the proportions, followed by mixing evenly.

100g of oats, 10g of bitter orange flowers, 30g of lotus leaves, 40g of dried orange peels

(2) Water was added and extraction was carried out. The mass ratio of the raw material to water is 1:40. Extraction was carried out at 80°C for 4h.

(3) The extract solution obtained in Step (2) was cooled to 30°C, filtered through 100-mesh sieve, and subjected to vacuum filtration after the residue was separated out. The extract solution was collected to obtain the composition.

In the vacuum filtration of Step (3), two layers of filter papers were paved in a Buchner funnel with 0.3cm to 0.6cm of diatomite in between.

## Example 4 Preparation of the slimming cream

Formula and dosage

**Table 3 Formula and dosage**

| Phases | INCI Name, herein "/" means "and" | Percentage (%) |
|---|---|---|
| Phase A | Cyclotetrasiloxane | 0.5% |
| | Cyclopentasiloxane | 0.5% |
| | Hydrogenated Polyisobutene | 2.5% |
| | Arachidyl Alcohol/Behenyl Alcohol/ArachidylGlucoside MONTANOV 202 (trade name of Arachidyl Alcohol, Behenyl Alcohol, Arachidyl Glucoside) | 3% |
| | Cetearyl Alcohol | 0.5% |
| Phase B | Water | As balance |
| | Glycerin | 3% |
| | PEG-8 | 1% |
| | Butanediol | 5% |
| | Dimethicone | 2% |
| | Caprylyl Methicone | 0.5% |
| | Acrylates/C10-30 alkyl acrylate crosspolymer | 1% |
| | Niacinamide | 2% |
| | Allantoin | 0.05% |

(continued)

| Phases | INCI Name, herein "/" means "and" | Percentage (%) |
|---|---|---|
| Phase C | Hydroxyethyl Acrylate/Sodium AcryloyldimethylTaurate Copolymer/ Squalane/Water/Polysorbate 60/Sorbitan Isostearate | 1.5% |
| Phase D | Aminomethyl Propanol | Adjust the pH to 6.5 |
| Phase E | Cafeisilane C | 6% |
| Phase E | Botany Extract | 5% |
| Phase E | Chondrus Crispus | 5% |
| Phase F | Phenoxyethanol | 0.2% |
| Phase F | Lavender Oil | 2% |

Preparation method of the cream:

(1) Phase A was added into an oil phase pot and heated to 80°C with stirring. The stirring rate was 40r/min and the heating rate was 1°C/min. The temperature was maintained constantly for 30min until Phase A was dissolved absolutely.

(2) Phase B was added into an aqueous phase pot and heated to 80°C with stirring (the stirring rate was 40r/min; the heating rate was 1°C/min). The temperature was maintained constantly for 30min.

(3) Emulsification: under condition of stirring (the stirring rate was 30r/min), the Phase A in the oil phase pot was transferred into an emulsifying pot, and then the Phase B was transferred into the emulsifying pot. Homogenous emulsification was performed under a condition of 2500r/min for 3min.

(4) Phase C was added into the emulsifying pot under stirring, and the stirring rate was 30r/min.

(5) Under the stirring with a stirring rate of 30r/min, the temperature was cooled to 40°C with a cooling rate of 1°C/min; Phase D, E and F were added in turns and stirred until evenly.

(6) Mixture was stirred and cooled to 40°C; a product was obtained through discharge.

## Example 5 Preparation of the slimming cream

Formula and dosage

**Table 4 Formula and dosage**

| Phases | INCI Name, herein "/" means "and" | Percentage (%) |
|---|---|---|
| Phase A | Cyclotetrasiloxane | 1.5% |
| Phase A | Cyclopentasiloxane | 3% |
| Phase A | Hydrogenated Polyisobutene | 1% |
| Phase A | Arachidyl Alcohol/Behenyl Alcohol/Arachidyl Glucoside MONTANOV 202 (trade name of Arachidyl Alcohol, Behenyl Alcohol, Arachidyl Glucoside) | 0.5% |
| Phase A | Cetearyl Alcohol | 1% |

(continued)

| Phases | INCI Name, herein "/" means "and" | Percentage (%) |
|---|---|---|
| Phase B | Water | As balance |
| | Glycerin | 1% |
| | PEG-8 | 5% |
| | Butanediol | 3% |
| | Dimethicone | 3% |
| | Caprylyl Methicone | 1.5% |
| | Acrylates/C10-30 alkyl acrylate crosspolymer | 0.2% |
| | Niacinamide | 1% |
| | Allantoin | 1% |
| Phase C | Hydroxyethyl Acrylate/Sodium AcryloyldimethylTaurate Copolymer/ Squalane/Water/Polysorbate 60/Sorbitan Isostearate | 0.1% |
| Phase D | Aminomethyl Propanol | Adjust the pH to 6.0 |
| Phase E | Cafeisilane C | 3.5% |
| | Botany Extract | 10% |
| | Chondrus Crispus | 0.5% |
| Phase F | Phenoxyethanol | 0.5% |
| | Lavender Oil | 1% |

Preparation method of the cream:

(1) Phase A was added into an oil phase pot and heated to 82°C with stirring. The stirring rate was 20r/min and the heating rate was 2°C/min. The temperature was maintained constantly for 35min until Phase A was dissolved absolutely.

(2) Phase B was added into an aqueous phase pot and heated to 82°C with stirring (the stirring rate was 20r/min; the heating rate was 2°C/min). The temperature was maintained constantly for 35min.

(3) Emulsification: under condition of stirring (the stirring rate was 40r/min), the Phase A in the oil phase pot was transferred into an emulsifying pot first, and then the Phase B was transferred into the emulsifying pot. Homogenous emulsification was performed under a condition of 3000r/min for 4min.

(4) Phase C was added into the emulsifying pot under stirring, and the stirring rate was 40r/min.

(5) Under the stirring with a stirring rate of 40r/min, the temperature was cooled to 45°C with a cooling rate of 2°C/min; Phase D, E and F were added in turns and stirred until evenly.

(6) Mixture was stirred and cooled to 35°C; a product was obtained through discharge.

**Example 6 Preparation of the slimming cream**

Formula and dosage

**Table 5 Formula and dosage**

| Phases | INCI Name, herein "/" means "and" | Percentage (%) |
|---|---|---|
| Phase A | Cyclotetrasiloxane | 3% |
| | Cyclopentasiloxane | 2% |
| | Hydrogenated Polyisobutene | 5% |
| | Arachidyl Alcohol/Behenyl Alcohol/Arachidyl Glucoside MONTANOV 202 (trade name of Arachidyl Alcohol, Behenyl Alcohol, Arachidyl Glucoside) | 2% |
| | Cetearyl Alcohol | 0.01% |
| Phase B | Water | As balance |
| | Glycerin | 5% |
| | PEG-8 | 3% |
| | Butanediol | 1% |
| | Dimethicone | 0.5% |
| | Caprylyl Methicone | 3% |
| | Acrylates/C10-30 alkyl acrylate crosspolymer | 2% |
| | Niacinamide | 0.1% |
| | Allantoin | 0.05% |
| Phase C | Hydroxyethyl Acrylate/Sodium AcryloyldimethylTaurate Copolymer/ Squalane/Water/Polysorbate 60/Sorbitan Isostearate | 3% |
| Phase D | Aminomethyl Propanol | Adjust the pH to 5.0 |
| Phase E | CafeisilaneC | 1% |
| | Botany extract | 1% |
| | Chondrus Crispus | 10% |
| Phase F | Phenoxyethanol | 1.1% |
| | Lavender Oil | 0.005% |

Preparation method of the cream:

(1) Phase A was added into an oil phase pot and heated to 85°C with stirring. The stirring rate was 60r/min and the heating rate was 1°C/min. The temperature was maintained constantly for 32min until Phase A was dissolved absolutely.

(2) Phase B was added into an aqueous phase pot and heated to 85°C with stirring (the stirring rate was 60r/min; the heating rate was 2°C/min). The temperature was maintained constantly for 32min.

(3) Emulsification: under condition of stirring (the stirring rate was 50r/min), the Phase A in the oil phase pot was transferred into an emulsifying pot first, and then the Phase B was transferred into the emulsifying pot. Homogenous emulsification was performed under a condition of 3500r/min for 5min.

(4) Phase C was added into the emulsifying pot under stirring, and the stirring rate was 50r/min.

(5) Under the stirring with a stirring rate of 50r/min, the temperature was cooled to 50°C with a cooling rate of 2°C/min; Phase D, E and F were added in turns and stirred until evenly.

(6) Mixture was stirred and cooled to 30°C; a product was obtained through discharge.

**Example 7 Efficacy experiment**

**I. Effect of the slimming composition prepared in Example 1, Example 2 and Example 3 on collagen synthesis of CCD-966SK cells.**

1.1 Experiment materials

Human skin fibroblast CCD-966SK, Food Industry Research Institute, Taiwan; MEM cell culture medium (Minimum Essential Medium, Earle's), fetal bovine serum, non-essential amino acid and sodium pyruvate, from Invitrogen Corporation; dimethyl sulfoxide, Sigma-Aldrich; WST-1 (water-soluble tetrazole salt), Abnova Corporation; Sircol Collagen Assay kit, Biocolor Ltd.; samples from the examples.

1.2 Equipment

BP3100S electronic scales, Sartorius Intec; SZCL Intelligent Temperature Controlling Heating Stirrer, Gongyi City, Yuhua Instrument Co., Ltd.; SH-III Water Circulation Type Vacuum Pump, Zhengzhou Great Wall Scientific Industrial and Trade Co., Ltd.; M-1815 TC $CO_2$ incubator, America; FJ-2017 liquid scintillation counter, CNNC Xi'An Nuclear Instrument Factory; Sigma 4K15 platform high speed refrigerated centrifuge, Sigma-Aldrich, America; ADVANTAGE Vacuum Freeze Drying Device, Virtis, America; Multiskan GO enzyme-linked analyzer, Thermo.

1.3 Experiment method

Human skin fibroblast CCD-966SK was cultured in MEM culture medium (containing 10% of FBS, 1% of penicillin-streptomycin, 1% of non-essential amino acid and 1% of sodium pyruvate) in an incubator with 5% of $CO_2$ at 37°C. Medium was changed every 2 to 3days. Effect on cell proliferation: CCD-966SK cells were seeded in 96-well plate at a density of $1 \times 10^4$ cell/well; the sample (prepared in complete MEM medium containing 10% of fetal bovine serum) was also added to reach a total volume of $100\mu L$ per well. The content of collagen was determined by the Collagen Assay Kit (Sircol Collagen assay).

1.4 Experiment Results

The effect of the slimming composition obtained in Example 1, Example 2 and Example 3 on the collagen synthesis of human skin fibroblast CCD-966SK was evaluated. There was no botanical extract in control group. The experiment results were shown in Table 6. The results showed that the botanical extracts obtained in Example 1, Example 2 and Example 3 significantly promoted the synthesis of collagen in human skin fibroblast CCD-966SK ($P < 0.05$).

**Table 6 Results**

| Sample | Concentration of collagen (mg/mL) |
|---|---|
| Control group | 0 |
| Example 1 | 0.02 |
| Example 2 | 0.03 |
| Example 3 | 0.03 |

**II. Effect of the slimming composition prepared in Example 1, Example 2 and Example 3 on leptin secretion from adipocyte.**

1. Preparation of proliferation culture medium (1000mL)

850mL of deionized water was added to dry D-MEM/F-12(1:1) powder; after the powder dissolved totally, 2.44g of $NaHCO_3$ and 3.15g of HEPES were added. The final volume was adjusted to 1000mL with water and pH was adjusted to 7.2 by 0.1N HCl and NaOH. The solution was sterilized by passing through $0.22\mu m$ filtration membrane, divided into aliquots and stored at 4°C for use. When it was used as proliferation culture medium, 10% of inactivated calf serum was added under sterile condition; when it was used as basal culture medium, 10% of inactivated fetal bovine serum was added under sterile condition.

2. Preparation of differentiation culture medium I (100mL)

Insulin (10ug/mL), dexamethasone (1μM) and IBMX (0.5mM) were added to the basal culture medium. Medium was sterilized by passing through 0.22μm filtration membrane, divided into aliquots and stored at 4°C for use. Differentiation culture medium I was prepared by adding 10% of inactivated fetal bovine serum and penicillin-streptomycin under sterile condition.

3. Preparation of differentiation culture medium II (100mL)

1mg of insulin was added to the basal culture medium to prepare 100mL of differentiation culture medium with a final concentration of insulin at 10μg/mL. The medium was sterilized by passing through 0.22μm filtration membrane, divided into aliquots and stored at 4°C for use. Differentiation culture medium II was prepared by adding 10% of inactivated fetal bovine serum under sterile condition.

4. Cell culture

3T3-L1 preadipocytes were cultured in the proliferation medium containing 10% of calf serum in an incubator with 5% of $CO_2$ at 37°C. The medium was changed every 2 to 3 days, and subculture was carried out when the cells reached 50%-60% confluence. The induction of differentiation was performed according to typical cocktail method. The cell density was adjusted to $2 \times 10^5$ cell/mL and seeded on 6-well plate or 24-well plate for subculture. The culture medium was changed every 2 to 3 days until the confluence of monolayer cells. The proliferation medium was changed to the basal culture medium containing 10% of fetal bovine serum for 2 days. The basal medium was replaced by the differentiation culture medium I for 48h, followed by the induction in differentiation culture medium II. After 48h, basal culture medium containing 10% of fetal bovine serum was used for the culture and changed every 48h. 7 days later, more than 90% of the cells have a round shape, filling with lipid droplet, that is, mature adipocytes after differentiation.

5. Detect the effect of different concentration of Act on adipokine through ELISA

Three parallel wells were provided in each group. Supernatants in cell culture well were collected on the tenth day and the content of leptin was detected according to the instruction of the kit. The control group was set. The samples were applied at the eighth day of differentiation.

The results of the effect of products prepared in Examples 1 to 3 on leptin secretion from mature adipocyte were shown in Table 7. As shown in Table 7, the products of Examples 1 to 3 promoted leptin secretion from mature adipocyte, resulting in the increase of leptin secretion.

**Table 7 Results**

| Sample | Content of leptin (ng) |
| --- | --- |
| Control Group | 12 |
| Example 1 | 16±2 |
| Example 2 | 17±1 |
| Example 3 | 18±1 |

**III. Experiment of anti-inflammatory effect of the slimming extract prepared in Example land slimming cream prepared in Example 4 on guinea pig pruritus model**

1. Experimental materials

1.1 Experimental samples:

Slimming extract prepared in Example 1, slimming cream prepared in Example 4

1.2 Experimental animals

Healthy guinea pig, SPF/VAF grade, half male and half female, weight 250±10g. Provided by Beijing Jinmuyang Experimental Animal Breeding Co., Ltd., Quality Certificate No. of the Animal: SCXK(Jing) 2010-0001. Raising conditions:

Farm of Beijing Jinmuyang Experimental Animal Breeding Co., Ltd.

1.3 Reagents

Histamine Phosphate purchased from Sigma-Aldrich

2. Experimental method

2.1 Experimental dosage

The test samples were topically applied at an amount of 0.15mL/cm$^2$, 0.1 mL/cm$^2$ and 0.05 mL/cm$^2$ as high dose group, medium dose group and low dose group, respectively. The model control group was topically applied with distilled water at an amount of 0.1mL/cm$^2$.

2.2 Histamine Phosphate itching method:

36 guinea pigs were divided into groups randomly: model control group, high dose group, medium dose group and low dose group. The fur on the back of the right hind foot was shaved the day before the experiment. The samples were applied respectively on the shaved skin evenly for 3 continuous days, while the model control group was applied with distilled water. On the third day of experiment, appropriate amount of histamine phosphate was accurately weighed and diluted in distilled water to gradual concentrations of 0.01%, 0.02%, 0.03%, 0.04%, 0.05%, 0.06%, 0.07%, 0.08%, 0.09% and 0.10% before use. The shaved area on the back of right hind foot of the guinea pig was abraded by coarse sand paper for an area about 1 cm$^2$. The sample was topically applied one more time. 10min later, 0.05mL of 0.01% histamine phosphate was dropped on the abraded area. Thereafter, 0.05mL histamine phosphate was dropped every 3min at a gradual concentrations of 0.01%, 0.02%, 0.03%, 0.04%..., until the guinea pig started licking the right hind foot. The itching threshold was set as the total amount of histamine phosphate causing the guinea pig to start licking its right hind foot. The itching thresholds of every group were calculated and the differences between the groups were measured.

3. Results

The results showed that after the application of histamine phosphate to cause itch, the guinea pigs showed behavior of licking their right hind feet. After the sample was applied on the itching area, the histamine phosphate itching threshold of the high dose group increased greatly, showing significant difference compared to the model control group ($P<0.05$). The results were shown in Table 8.

**Table 8 Effect of the sample on histamine phosphate itching reaction of guinea pigs ($x\pm s$, n=6)**

| Group | Dosage(ml/cm$^2$) | Itching Threshold ($\mu$g) |
|---|---|---|
| Model Control Group | 0.1 | 30.83±11.14 |
| Example 4 High Dose Group | 0.15 | 58.83±24.17* |
| Example 1 High Dose Group | | 50.12±20.13 |
| Example 4 Middle Dose Group | 0.1 | 45.67±20.17 |
| Example 1 Middle Dose Group | | 41.55±21.11 |
| Example 4 Low Dose Group | 0.05 | 35.50±14.75 |
| Example1 Low Dose Group | | 29.97±13.56 |
| Note: Compared with the model control group, *P<0.05. | | |

4. Conclusions

The slimming extract prepared in Example 1 and the slimming cream prepared in Example 4 can effectively increase the itching threshold of the itching caused by histamine phosphate, proving its effect of relieving dermatitis and stopping pruritus.

The slimming compositions prepared in Example 2 and Example 3 and the slimming cream prepared in Example 5 and Example 6 were used to repeat the experiment. Similar results were obtained, which have no significant difference with that of the slimming extract in Example 1 and the slimming cream in Example 4 (P>0.05).

**IV. Skin repairing function evaluation of the preparations prepared in Example 1 and Example 4**

1. Experimental materials

1.1 Experimental samples

Slimming extract prepared in Example 1, slimming cream prepared in Example 4.

1.2 Experimental animals

36 healthy guinea pigs, SPF/VAF grade, half male and half female, weight 250 $\pm$ 10g. Provided by Beijing Jinmuyang Experimental Animal Breeding Co., Ltd., Quality Certificate No. of the Animal: SCXK (Jing) 2010-0001. Raising conditions: Farm of Beijing Jinmuyang Experimental Animal Breeding Co., Ltd.

1.3 Reagents and equipment

Acetone and ether from Beijing Chemical Works
Skin moisture analyzer

2. Experimental method

2.1 Experimental dosage

The test samples were topically applied at an amount of $0.15g/cm^2$, $0.1g/cm^2$ and $0.05g/cm^2$ as high dose group, medium dose group and low dose group, respectively. The control group was topically applied with distilled water at an amount of $0.1mL/cm^2$.

2.2

36 guinea pigs were divided into groups randomly: blank control group, model control group, high dose group, medium dose group and low dose group. The fur on the nape of guinea pig was shaved at an area about $2\times2cm^2$ the day before the experiment. Except for the blank control group, $150\mu L$ of a mixture (acetone:ether=1:1) was dropped on the shaved skin in all other groups. 10min later, the corresponding samples were applied respectively on the shaved areas, twice a day for 5 days. Distilled water was applied to the blank control group and model control group on the shaved area. On the fifth day, the moisture content of the shaved skin was tested 20min after the application of the sample. The differences between the groups were measured and the water loss protection rate was calculated.
The effect of the sample on skin moisture content in the skin dehydration model provided experimental basis for evaluating the repairing function of the sample to the allergic skin.
Water loss protection rate(%) = (the skin moisture content of the sample group - the skin moisture content of the model group) / the skin moisture content of the blank control group $\times$ 100

3. Results

The results showed that after dehydration of guinea pig skin, the skin dehydration rate of the model control group was 50.75%. After application of the samples, the slimming composition prepared in Example 1 and the slimming cream prepared in Example 4 greatly increased the moisture content of the guinea pig skin, showing significant difference to that of the model control group (P < 0.05). The results were shown in Table 9.

**Table 9 Repairing function for allergic skin (x$\pm$s, n=6**

| Group | Dosage (g/cm$^2$) | Water loss protection rate (%) |
|---|---|---|
| Blank control group | 0.1 | -- |
| Model control group | 0.1 | -- |

(continued)

| Group | Dosage (g/cm$^2$) | Water loss protection rate (%) |
|---|---|---|
| High dose group of Example 4 | 0.15 | 32.08* |
| High dose group of Example 1 | | 29.03 |
| Medium dose group of Example 4 | 0.1 | 25.63 |
| Medium dose group of Example 1 | | 23.53 |
| Low dose group of Example 4 | 0.05 | 18.60 |
| Low dose group of Example 4 | | 17.10 |

4. Conclusion

The slimming composition prepared in Example 1 and the slimming cream prepared in Example 4 have obvious skin repairing function.

The slimming preparations prepared in Example 2 and Example 3 and the slimming cream prepared in Example 5 and Example 6 were used to repeat the above experiment. Similar effects were obtained, which have no significant difference with that of the slimming exact prepared in Example 1 and the slimming cream prepared in Example 4 (P > 0.05), indicating that the preparations prepared in the present disclosure have good repairing function.

**V. Efficacy evaluation of the preparations prepared in Example 1 and Example 4 on removing redness and swelling**

1. Experimental materials

1.1 Experimental samples

Slimming extract prepared in Example 1, slimming cream prepared in Example 4

1.2 Experimental animals

36 SD rats, SPF/VAF grade, half male and half female, weight $180 \pm 10$g. Provided by Beijing Jinmuyang Experimental Animal Breeding Co., Ltd., Quality Certificate No. of the Animal: SCXK (Jing) 2010-0001. Raising conditions: Farm of Beijing Jinmuyang Experimental Animal Breeding Co., Ltd.

1.3 Reagents and equipment

Anti-DNP IgE, DNP-HAS and Evans Blue, Sigma-Aldrich Product; acetone, Beijing Chemical Works; RT-6000 micro-plate reader, Rayto Life and Analytical Sciences Co.,Ltd..

2. Experimental method

2.1 Experimental dosage

The test samples were topically applied at an amount of 0.15g/cm$^2$, 0.1g/cm$^2$ and 0.05g/cm$^2$ as high dose group, medium dose group and low dose group, respectively. The blank control group and the model control group were topically applied with distilled water at an amount of 0.1g/cm$^2$.

2.2 Skin allergy test

36 rats were housed in cages at room temperature with free access to food and water for 5 day's adaptive feeding. The rats were randomly divided into groups by weight: blank control group, model control group, high dose group, medium dose group and low dose group, 6 rats per group. The fur on the back was shaved. Except for the blank control group, 0.05$\mu$g (0.5$\mu$l) anti-DNP IgE was subcutaneously injected in 3 spots on the back of the rat. 48h later, 100 $\mu$g (100 $\mu$l) DNP-HAS containing 4% of Evans Blue was injected through the caudal vein of the rats. 1h before the caudal vein injection of DNP-HAS, samples were applied to 2cm$^2$ area surrounding the injection spot of anti-DNP IgE on the back of the rat. The rats were sacrificed 30min after intravenous injection of DNP-HAS. The blue-dyed skin was cut off and emerged in acetone-physiological saline mixed solution (1:1) for 24h. The solution was subjected to centrifuge to separate the supernatant, and the optical density was detected by spectrophotometer at 620nm. Evans Blue solution was used to establish the standard curve. The pigment content of the back skin of the rat and the inhibitory rate of PCA reaction were calculated. The inhibitory rate is calculated by the formula as follow:

$$\text{Inhibitory rate (\%)} = (\text{pigment content of model control group} - \text{pigment content of treatment group})/(\text{pigment content of model control group}) \times 100$$

3. Results

As shown in the results, blue-dyed skin showed up on local skin of the rat after sensitization. The pigment content of the blue-dyed rat skin was dramatically decreased after the application of the samples, demonstrating extremely significant differences or significant differences compared to the model control group ($P < 0.01$, $P < 0.05$). The results were shown in Table 10.

**Table 10 Effect of the sample on PCA reaction in the rats**

| Group | Dosage (g/cm$^2$) | Inhibitory rate(%) |
|---|---|---|
| Blank group | 0.1 | -- |
| Model group | 0.1 | -- |
| High dose group of Example 4 | 0.15 | 10.89* |
| High dose group of Example 1 | | 9.11 |
| Medium dose group of Example 4 | 0.1 | 8.95 |
| Medium dose group of Example 1 | | 7.01 |
| Low dose group of Example 4 | 0.05 | 6.02 |
| Low dose group of Example 1 | | 5.48 |

4. Conclusion

The slimming extract prepared in Example 1 and the slimming cream prepared in Example 4 greatly decreased the pigment content of the rat blue-dyed skin after sensitization, indicating that the preparations of the present disclosure have potential function of removing redness and swelling caused by exogenous stimulation.
The preparations prepared in Example 2, Example 3, Example 5 and Example 6 were used to repeat the above experiment. Similar effects were obtained, which have no significant difference with that of the slimming exact prepared in Example 1 and the slimming cream prepared in Example 4 ($P > 0.05$).

**VI. Evaluation of anti-fatigue efficacy of the composition prepared in Example 1 and cream prepared in Example 4**

In recent years, chronic fatigue caused by being sedentary and excessive mental work has become one of the common problems for office workers. The main symptoms are pain and discomfort of shoulders and neck, which seriously affects people's work efficiency and life quality. The anti-fatigue efficacy of the samples prepared in the present examples was evaluated according to the following method.
Samples: the botanic composition prepared in Example land the cream prepared in Example 4.
Subject: 30 office workers who have symptom of neck pain were divided into two groups randomly, 15 per group. The composition prepared in Example 1 and the cream prepared in Example 4 were used for trial.
Application method: smear and massage the samples on soreness area such as neck twice a day for 4 weeks, in the morning and at night, respectively.

Evaluation standard:

significant effect: disappear of the pain ; relieved symptom: relieving of the pain; no effect: no significant difference between before and after; effective: significant improvement and the relieving of symptoms.

The statistics of the results from 30 subjects were shown in Table 11.

**Table 11 Evaluation of anti-fatigue effect of the sample**

|  | Significant | Relieved | No effect | Effective rate (%) |
|---|---|---|---|---|
| Example 1 | 2 | 6 | 7 | 53% |
| Example 4 | 3 | 7 | 5 | 67% |

The evaluation results showed that the composition and cream prepared in the present disclosure have effects on relieving soreness and anti-fatigue. The samples prepared in Example 2, Example 3, Example 5 and Example6 were used to repeat the above experiment. Similar effects were obtain, which have no significant difference with that of the slimming exact prepared in Example 1 and the slimming cream prepared in Example 4 ($P > 0.05$).

**VII. Evaluation of blood circulation-promoting effect of the preparations prepared in Example 1 and Example 4**

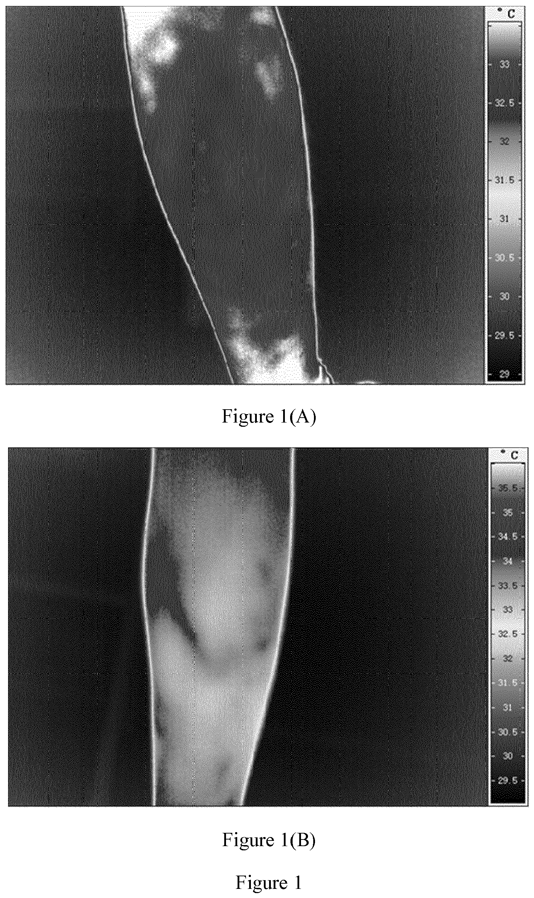

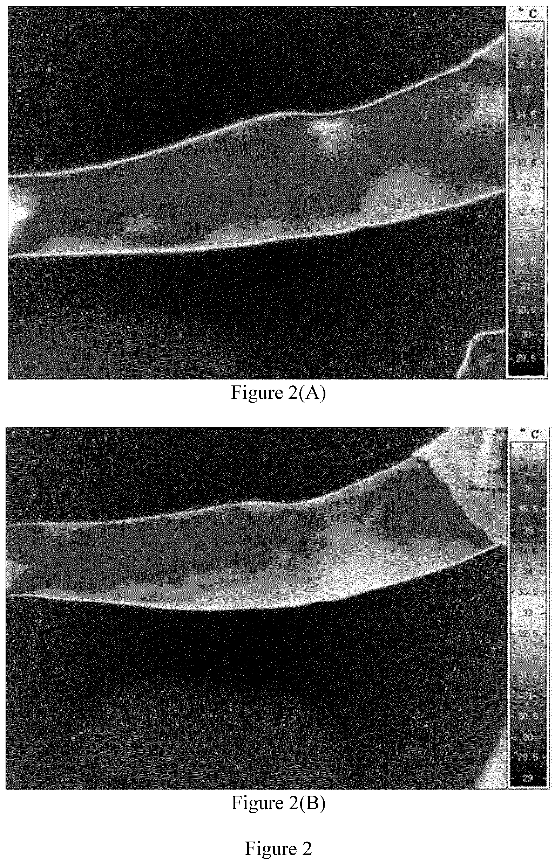

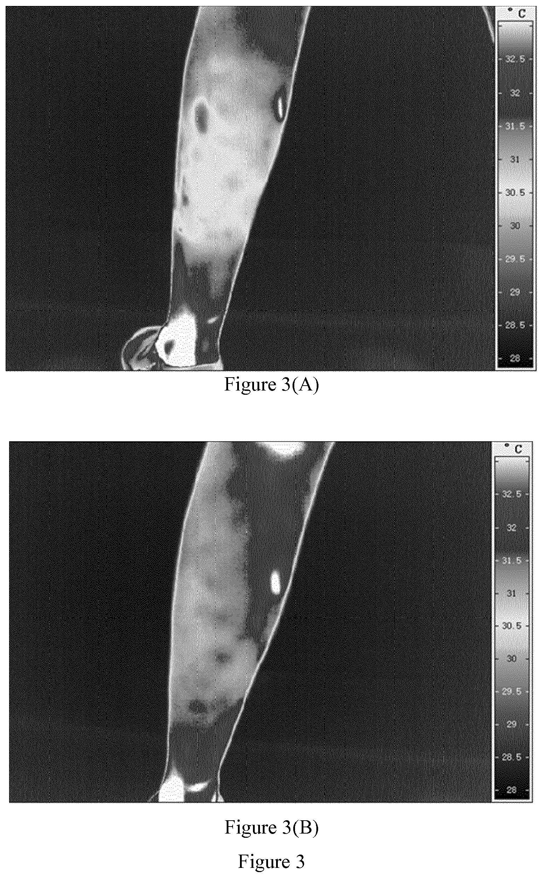

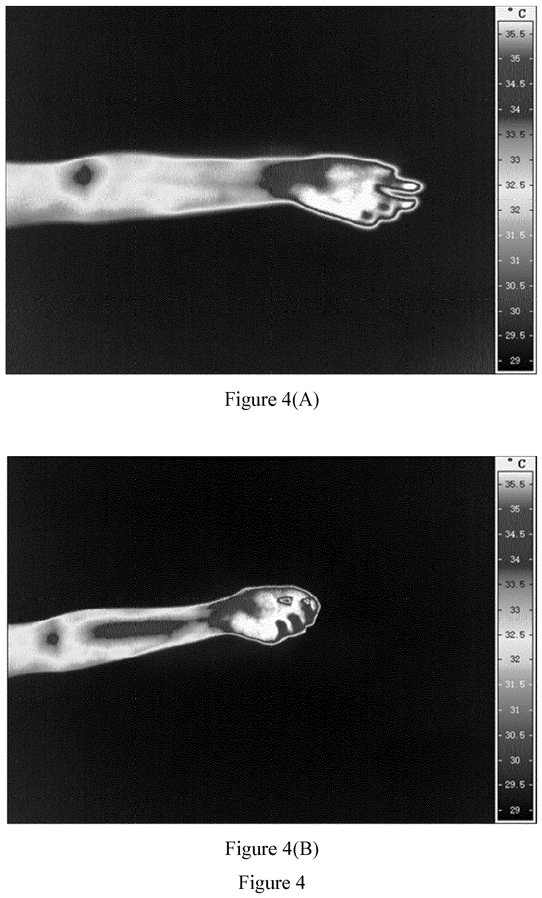

Sample: the botanical extract obtained in Example 1, the cream obtained in Example 4.
Application method: smear the samples on shank and forearm, twice a day for 4 weeks, in the morning and at night, respectively.
Evaluation method: the blood circulation-promoting effect of the skin care product can be estimated by infrared thermography. The infrared thermography of skin can detect the blood flow velocity and temperature of the test area, which indicates the conditions of blood circulation. Fast blood flow and high temperature means a good condition. The blood circulation-promoting effect is evaluated by comparing the infrared thermography before and after the use of samples. Figure 1 and Figure 2 showed the infrared thermography of shank and forearm that were applied with the composition in Example 1; Figure 3 and Figure 4 showed the infrared thermography of shank and forearm that were applied with the composition in Example 4. As shown in the skin infrared thermography, the blood flow velocity of the subject was increased after using the samples (see Figure 1(B), Figure 2(B), Figure 3(B), and Figure 4(B)). In the figures, the test area with lighter color indicates the area with fast blood flow.
The infrared thermography demonstrates that the samples obtained in Example 1 and Example 4 have good efficacy on promoting blood circulation. The samples obtained in Example 2, Example 3, Example 5 and Example 6 were used to repeat the experiment. Similar results as above were obtained, which have no significant difference with that of the slimming extract prepared in Example 1 and the slimming cream prepared in Example 4 ($P>0.05$).

**Claims**

1. A composition, comprising the following raw materials in parts by mass:

| | |
|---|---|
| Oats | 5 to 10 parts |
| Bitter orange flowers | 1 to 3 parts |
| Lotus leaves | 1 to 3 parts |
| Dried orange peels | 3 to5 parts |

2. A method for preparing the composition according to claim 1, comprising: pulverizing the desired amount of raw materials according to the formula, extracting with water; the temperature for water extraction is from 61°C to 100°C, the duration of water extraction is from 1h to 4h, the mass ratio of the raw materials to water is from 1:21 to 1:40.

3. The composition according to claim 1, or the composition prepared by the method according to claim 2 for use in slimming.

4. The composition according to claim 1, or the composition prepared by the method according to claim 2 for use in skin care.

5. The composition according to claim 1, or the composition prepared by the preparation method according to claim 2 for use in treating pruritus, repairing skin, removing redness and swelling, anti-fatigue, promoting blood circulation and promoting collagen synthesis.

6. A skin care product, comprising the composition according to claim 1 or the composition prepared by the method according to claim 2 and adjuvant acceptable in skin care products.

7. The skin care product according to claim 6, wherein the dosage form of the skin care product is paste, cream, essence, toner, emulsion or spray.

8. A method for preparing the skin care product according to claim 6 or claim 7, wherein the skin care product is made from the following raw materials in parts by mass:

| Phases | INCI Name | Percentage (wt%) |
|---|---|---|
| Phase | Cyclotetrasiloxane | 0.5~3 wt% |
| A | Cyclopentasiloxane | 0.5~3 wt% |
| | Hydrogenated Polyisobutene | 1~5wt% |
| | Arachidyl Alcohol | 0.5~3wt% |
| | Behenyl Alcohol | |
| | Arachidyl Glucoside | |
| | Cetearyl Alcohol | 0.01~1wt% |
| Phase B | Water | As balance |
| | Glycerin | 1~5 wt% |
| | PEG-8 | 1~5wt% |
| | Butanediol | 1~5 wt% |
| | Dimethicone | 0.5~3wt% |
| | Caprylyl Methicone | 0.5~3wt% |
| | Acrylates/C10-30 alkyl acrylate crosspolymer | 0.2~2wt% |
| | Niacinamide | 0.1~2wt% |
| | Allantoin | 0.05~1 wt% |
| Phase C | Hydroxyethyl Acrylate/Sodium Acryloyldimethyl Taurate Copolymer/ Squalane/Water/Polysorb ate- 60 /Sorbitan Isostearate | 0.1~3wt% |
| Phase D | Aminomethyl Propanol | Adjust the pH to 5.0~6.5 |
| Phase E | Cafeisilane C | 1~6wt% |
| | Composition of claim 1 | 1~10wt% |
| | Chondrus Crispus | 0.5~10wt% |
| Phase F | Phenoxyethanol | 0.2~1.1wt% |
| | Lavender Oil | 0.005~2 wt% |

and wherein,
the method comprises steps as follows:

Step 1: heating and stirring Phase A to obtain a first product;
Step 2: heating and stirring Phase B to obtain a second product;
Step 3: mixing the first product and the second product, emulsifying to obtain a third product;
Step 4: mixing Phase C with the third product to obtain a fourth product;
Step 5: stirring and cooling the fourth product, adding Phase D, Phase E and Phase F in turns, mixing and cooling to obtain a product.

9. The method according to claim 8, wherein
the heating temperature of Step 1 is from 80°C to 85°C, the heating rate is from 1°C/min to 2°C/min, the heating duration is from 30min to 35min, the stirring rate in Step 1 is from 20r/min to 60r/min;
the heating temperature of the Step 2 is from 80°C to 85°C, the heating rate is from 1°C/min to 2°C/min, the heating duration is from 30min to 35min, the stirring rate in Step 1 is from 20r/min to 60r/min;
the stirring rate of the mixing of Step 3 is from 30r/min to 50r/min; the emulsification is a homogenous emulsification under a condition of 2500r/min to 3500r/min for 3min to 5min;
the stirring rate of the mixing of Step 4 is from 30r/min to 50r/min;
the stirring rate of Step 5 is from 30r/min to 50r/min, the cooling temperature is from 40°C to 50°C, the cooling rate is from 1°C/min to 2°C/min;
the cooling temperature of Step 5 is 30°C to 40°C.

10. A skin care product, wherein the skin care product is prepared by the method according to claim 8 or claim 9.

**Patentansprüche**

1.  Zusammensetzung, umfassend die folgenden Rohstoffe in Massenteilen:

    | | |
    |---|---|
    | Hafer | 5 bis 10 Teile |
    | Bitterorangenblüten | 1 bis 3 Teile |
    | Lotusblätter | 1 bis 3 Teile |
    | Getrocknete Orangenschalen | 3 bis 5 Teile |

2.  Verfahren zur Herstellung der Zusammensetzung nach Anspruch 1, umfassend: Pulverisieren der gewünschten Menge von Rohstoffen gemäß der Formel, Extrahieren mit Wasser; wobei die Temperatur für die Wasser-Extraktion von 61°C bis 100°C beträgt, die Dauer der Wasser-Extraktion von 1 Std bis 4 Std beträgt, das Massenverhältnis der Rohstoffe zu Wasser von 1:21 bis 1:40 beträgt.

3.  Zusammensetzung nach Anspruch 1 oder durch das Verfahren nach Anspruch 2 hergestellte Zusammensetzung zur Verwendung bei der Gewichtsabnahme.

4.  Zusammensetzung nach Anspruch 1 oder durch das Verfahren nach Anspruch 2 hergestellte Zusammensetzung zur Verwendung in der Hautpflege.

5.  Zusammensetzung nach Anspruch 1 oder durch das Herstellungsverfahren nach Anspruch 2 hergestellte Zusammensetzung zur Verwendung bei der Behandlung von Juckreiz, Reparatur von Haut, Beseitigung von Röte und Schwellungen, Bekämpfung von Ermüdung, Förderung der Durchblutung und Förderung der Kollagensynthese.

6.  Hautpflegeprodukt, umfassend die Zusammensetzung nach Anspruch 1 oder die durch das Verfahren nach Anspruch 2 hergestellte Zusammensetzung und ein in Hautpflegeprodukten verträgliches Adjuvans.

7.  Hautpflegeprodukt nach Anspruch 6, wobei die Dosisform des Hautpflegeprodukts eine Paste, eine Creme, eine Essenz, ein Toner, eine Emulsion oder ein Spray ist.

8.  Verfahren zur Herstellung des Hautpflegeprodukts nach Anspruch 6 oder Anspruch 7, wobei das Hautpflegeprodukt aus den folgenden Rohstoffen in Massenteilen gefertigt wird:

| Phasen | INCI-Name | Prozentanteil (Gew.-%) |
|---|---|---|
| Phase A | Cyclotetrasiloxan | 0,5~3 Gew.-% |
| | Cyclopentasiloxan | 0,5~3 Gew.-% |
| | Hydriertes Polyisobuten | 1~5 Gew.-% |
| | Arachidylalkohol | 0,5~3 Gew.-% |
| | Behenylalkohol | |
| | Arachidylglucosid | |
| | Cetearylalkohol | 0,01~1 Gew.-% |
| Phase B | Wasser | Als Rest |
| | Glycerin | 1~5 Gew.-% |
| | PEG-8 | 1~5 Gew.-% |
| | Butandiol | 1~5 Gew.-% |
| | Dimethicon | 0,5~3 Gew.-% |
| | Caprylylmethicon | 0,5~3 Gew.-% |
| | Acrylate/C10-30-Alkylacrylat-Crosspolymer | 0,2~2 Gew.-% |
| | Niacinamid | 0,1~2 Gew.-% |
| | Allantoin | 0,05~1 Gew.-% |
| Phase C | Hydroxyethylacrylat/-Natriumacryloyldimethyltaurat-Copolymer/Squalan/Wasser/-Polysorbat-60/Sorbitanisostearat | 0,1~3 Gew.-% |
| Phase D | Aminomethylpropanol | pH-Wert auf 5,0~6,5 einstellen |
| Phase E | Cafeisilan C | 1~6 Gew.-% |
| | Zusammensetzung von Anspruch 1 | 1~10 Gew.-% |
| | Chondrus crispus | 0,5~10 Gew.-% |
| Phase F | Phenoxyethanol | 0,2~1,1 Gew.-% |
| | Lavendelöl | 0,005~2 Gew.-% |

und wobei

das Verfahren Schritte wie folgt umfasst:

Schritt 1: Erhitzen und Rühren von Phase A, um ein erstes Produkt zu erhalten;
Schritt 2: Erhitzen und Rühren von Phase B, um ein zweites Produkt zu erhalten;
Schritt 3: Mischen des ersten Produkts und des zweiten Produkts, Emulgieren, um ein drittes Produkt zu erhalten;
Schritt 4: Mischen von Phase C mit dem dritten Produkt, um ein viertes Produkt zu erhalten;
Schritt 5: Rühren und Abkühlen des vierten Produkts, Zugeben von Phase D, Phase E und Phase F der Reihe nach, Mischen und Abkühlen, um ein Produkt zu erhalten.

**9.** Verfahren nach Anspruch 8, wobei

die Erhitzungstemperatur von Schritt 1 von 80°C bis 85°C beträgt, die Erhitzungsrate von 1°C/min bis 2°C/min beträgt, die Erhitzungsdauer von 30 min bis 35 min beträgt, die Rührgeschwindigkeit in Schritt 1 von 20 U/min bis 60 U/min beträgt;

die Erhitzungstemperatur des Schritts 2 von 80°C bis 85°C beträgt, die Erhitzungsrate von 1°C/min bis 2°C/min beträgt, die Erhitzungsdauer von 30 min bis 35 min beträgt, die Rührgeschwindigkeit in Schritt 1 von 20 U/min bis 60 U/min beträgt;

die Rührgeschwindigkeit des Mischens von Schritt 3 von 30 U/min bis 50 U/min beträgt; die Emulgierung eine homogene Emulgierung unter einer Bedingung von 2500 U/min bis 3500 U/min für 3 min bis 5 min ist;

die Rührgeschwindigkeit des Mischens von Schritt 4 von 30 U/min bis 50 U/min beträgt; die Rührgeschwindigkeit von Schritt 5 von 30 U/min bis 50 U/min beträgt, die Abkühltemperatur von 40°C bis 50°C beträgt, die Abkühlrate von 1°C/min bis 2°C/min beträgt;
die Abkühltemperatur von Schritt 5 von 30°C bis 40°C beträgt.

**10.** Hautpflegeprodukt, wobei das Hautpflegeprodukt durch das Verfahren nach Anspruch 8 oder Anspruch 9 hergestellt wird.

**Revendications**

**1.** Composition, comprenant les matières premières suivantes en parties en masse :

| | |
|---|---|
| Avoine | 5 à 10 parties |
| Fleurs d'oranger amère | 1 à 3 parties |
| Feuilles de lotus | 1 à 3 parties |
| Zestes séchés d'orange | 3 à 5 parties |

**2.** Méthode de préparation de la composition selon la revendication 1, comprenant : la pulvérisation de la quantité désirée de matières premières selon la formule, l'extraction avec de l'eau ; la température de l'extraction à l'eau va de 61 °C à 100 °C, la durée de l'extraction à l'eau va de 1 h à 4 h, le rapport en masse des matières premières sur l'eau va de 1/21 à 1/40.

**3.** Composition selon la revendication 1, ou composition préparée par la méthode selon la revendication 2 pour l'utilisation dans l'amincissement.

**4.** Composition selon la revendication 1, ou composition préparée par la méthode selon la revendication 2 pour l'utilisation dans les soins cutanés.

**5.** Composition selon la revendication 1, ou composition préparée par la méthode de préparation selon la revendication 2 pour l'utilisation dans le traitement du prurit, la réparation de la peau, l'élimination des rougeurs et gonflements, contre la fatigue, pour favoriser la circulation sanguine et favoriser la synthèse de collagène.

**6.** Produit de soins cutanés, comprenant la composition selon la revendication 1 ou la composition préparée par la méthode selon la revendication 2 et un adjuvant acceptable dans les produits de soins cutanés.

**7.** Produit de soins cutanés selon la revendication 6, dans lequel la forme de dosage du produit de soins cutanés est la pâte, la crème, l'essence, le toner, l'émulsion ou le spray.

**8.** Méthode de préparation du produit de soins cutanés selon la revendication 6 ou la revendication 7, dans laquelle le produit de soins cutanés est fabriqué à partir des matières premières suivantes en parties en masse :

| Phases | Nom INCI | Pourcentage (% en poids) |
|---|---|---|
| | Cyclotetrasiloxane | 0,5 ~ 3 % en poids |
| | Cyclopentasiloxane | 0,5 ~ 3 % en poids |
| | Hydrogenated Polyisobutene | 1 ~ 5 % en poids |
| Phase A | Arachidyl Alcohol | |
| | Behenyl Alcohol | 0,5 ~ 3 % en poids |
| | Arachidyl Glucoside | |
| | Cetearyl Alcohol | 0,01 ~ 1 % en poids |

(suite)

| Phases | Nom INCI | Pourcentage (% en poids) |
|---|---|---|
| Phase B | Aqua | Pour le reste |
| | Glycerin | 1 ~ 5 % en poids |
| | PEG-8 | 1 ~ 5 % en poids |
| | Butanediol | 1 ~ 5 % en poids |
| | Dimethicone | 0,5 ~ 3 % en poids |
| | Caprylyl Methicone | 0,5 ~ 3 % en poids |
| | Acrylates/C10-30 alkyl acrylate crosspolymer | 0,2 ~ 2 % en poids |
| | Niacinamide | 0,1 ~ 2 % en poids |
| | Allantoin | 0,05 ~ 1 % en poids |
| Phase C | Hydroxyethyl Acrylate/ Sodium Acryloyldimethyl Taurate Copolymer/Squalane/Aqua/ Polysorbate-60/Sorbitan Isostearate | 0,1 ~ 3 % en poids |
| Phase D | Aminomethyl Propanol | Ajuster le pH à 5,0 ~ 6, 5 |
| Phase E | Cafeisilane C | 1 ~ 6 % en poids |
| | Composition selon la revendication 1 | 1 ~ 10 % en poids |
| | Chondrus Crispus | 0,5 ~ 10 % en poids |
| Phase F | Phenoxyethanol | 0,2 ~ 1,1 % en poids |
| | Lavender Oil | 0,005 ~ 2 % en poids |

et dans laquelle
la méthode comprend les étapes suivantes :

Étape 1 : chauffage et agitation de la phase A pour obtenir un premier produit ;
Étape 2 : chauffage et agitation de la phase B pour obtenir un deuxième produit ;
Étape 3 : mélange du premier produit et du deuxième produit, émulsification pour obtenir un troisième produit ;
Étape 4 : mélange de la phase C avec le troisième produit pour obtenir un quatrième produit ;
Étape 5 : agitation et refroidissement du quatrième produit, addition de la phase D, de la phase E et de la phase F tour à tour, mélange et refroidissement pour obtenir un produit.

9. Méthode selon la revendication 8, dans laquelle
la température de chauffage de l'étape 1 va de 80 °C à 85 °C, la vitesse de chauffage va de 1 °C/min à 2 °C/min, la durée de chauffage va de 30 min à 35 min, la vitesse d'agitation à l'étape 1 va de 20 t/min à 60 t/min ;
la température de chauffage de l'étape 2 va de 80 °C à 85 °C, la vitesse de chauffage va de 1 °C/min à 2 °C/min, la durée de chauffage va de 30 min à 35 min, la vitesse d'agitation à l'étape 1 va de 20 t/min à 60 t/min ;
la vitesse d'agitation du mélange de l'étape 3 va de 30 t/min à 50 t/min ; l'émulsification est une émulsification homogène dans une condition de 2500 t/min à 3500 t/min pendant 3 min à 5 min ;
la vitesse d'agitation du mélange de l'étape 4 va de 30 t/min à 50 t/min ; la vitesse d'agitation de l'étape 5 va de 30 t/min à 50 t/min, la température de refroidissement va de 40 °C à 50 °C, la vitesse de refroidissement va de 1 °C/min à 2 °C/min ;
la température de refroidissement de l'étape 5 va de 30 °C à 40 °C.

10. Produit de soins cutanés, dans lequel le produit de soins cutanés est préparé par la méthode selon la revendication 8 ou la revendication 9.

Figure 1(A)

Figure 1(B)

Figure 1

Figure 2(A)

Figure 2(B)

Figure 2

Figure 3(A)

Figure 3(B)

Figure 3

Figure 4(A)

Figure 4(B)

Figure 4